# EUROPEAN PATENT APPLICATION

(11) **EP 4 083 678 A1**
(43) Date of publication of application: **02.11.2022**
(21) Application number: 20907639.7
(22) Date of filing: 21.12.2020
(51) Int. Cl.: G02B 23/24, A61B 1/00, A61B 1/01, A61B 1/06

(54) **ENDOSCOPE AND ENDOSCOPE SYSTEM**

(30) Priority: 26.12.2019 JP 2019236272
(71) Applicant: FUJIFILM Corporation, Tokyo 106-8620 (JP)
(72) Inventor: TATSUTA, Takeichi, Ashigarakami-gun, Kanagawa 258-8538 (JP); SUGIZAKI, Makoto, Ashigarakami-gun, Kanagawa 258-8538 (JP); INOMATA, Kazuaki, Ashigarakami-gun, Kanagawa 258-8538 (JP)
(74) Representative: Hughes, Andrea Michelle
(86) International application number: PCT/JP2020/047673
(87) International publication number: WO 2021/132153

(57) **Abstract**

There are provided an endoscope and an endoscope system that can fix the position of an insertion part and allow an object to be observed to be measured with high accuracy. A balloon (19) is attachably and detachably mounted on an insertion part (12a) of an endoscope. A distal end part (12d) provided at a distal end of the insertion part (12a) includes an image pickup optical system (21), an illumination optical system (22), and a beam light-emitting unit (23). A subject is irradiated with measurement light by the beam light-emitting unit (23). It is possible to recognize the position of a spot from a subject image, and to measure an observation distance to an object to be observed, the size of the object to be observed, and the like.

## Description

### BACKGROUND OF THE INVENTION

### 1. Field of the Invention

The present invention relates to an endoscope and an endoscope system that emits measurement light.

### 2. Description of the Related Art

An observation distance between an endoscope and an object to be observed, the size of the object to be observed, the three-dimensional shape of the object to be observed, or the like has been acquired in the field of endoscopes. For example, in WO2018/051680A, an object to be observed is irradiated with measurement light from an irradiation window provided at the distal end part of an endoscope and a spot is formed on the object to be observed. Accordingly, in a processor device for an endoscope, the position of the spot is specified from a subject image that is obtained from the image pickup of the object to be observed. Then, an observation distance from the position of the spot can be measured.

Further, in WO2018/051680A, concentric circular measurement markers are superimposed and displayed on the subject image to center on the spot. The sizes of the measurement markers change to correspond to the position of the spot. Accordingly, the size of the object to be observed can be measured from the comparison of the measurement markers and the object to be observed.

On the other hand, stripe-pattern light is used in JP2016-198304A as measurement light. In a case where stripe-pattern light is used as measurement light, the period of the stripe-pattern light changes depending on a distance from a subject. Accordingly, the subject is irradiated with the stripe-pattern light plural times while the period or phase of the stripe-pattern light is shifted by a liquid crystal shutter, a calculation is made on the basis of a plurality of subject images obtained by shifting the period or phase, and the three-dimensional shape of the subject is measured.

Furthermore, grid-pattern measurement light is used in JP2017-217215A as measurement light emitted from a distal end part of an endoscope. Accordingly, the three-dimensional shape of a subject is measured according to the state of deformation of a grid pattern in a case where an object to be observed is irradiated with the grid-pattern measurement light.

### SUMMARY OF THE INVENTION

However, in a case where an endoscope is used for medical treatment, the position of an insertion part of the endoscope may be shifted due to the body motion, heartbeat, and pulsation of a patient. In the endoscopes disclosed in WO2018/051680A, JP2016-198304A, and JP2017-217215A, an observation distance, the size and three-dimensional shape of an object to be observed, and the like cannot be measured with high accuracy in a case where the position of the insertion part is shifted. That is, in a case where the position of the insertion part is shifted after an object to be observed that a doctor wants to measure is irradiated with measurement light, the position of a portion irradiated with the measurement light is also shifted. For this reason, measurement cannot be performed with high accuracy on the basis of a spot or the like formed on the object to be observed. Particularly, in a case where a calculation is to be made on the basis of a plurality of subject images obtained by shifting the period or phase of the measurement light as in JP2016-198304A, not an actual object but a secondarily generated image (referred to as the artifact of a moving subject) may be formed in a case where the position of the insertion part is shifted during the irradiation using the measurement light.

The present invention has been made in consideration of the above-mentioned circumstances, and an object of the present invention is to provide an endoscope and an endoscope system that can fix the position of an insertion part and allow an object to be observed to be measured with high accuracy.

An endoscope according to an aspect of the present invention comprises an insertion part to be inserted into an object to be examined, a distal end part, a bendable part, an emitting unit, an image pickup optical system, and a fixing member. The distal end part is provided. The bendable part is connected to a proximal end of the distal end part and changes an orientation of the distal end part by being operated to be bent. The emitting unit is disposed in the distal end part and emits measurement light. The image pickup optical system is disposed in the distal end part. In a case where the image pickup optical system is used to observe the inside of the object to be examined, the fixing member fixes the position of the insertion part with respect to the object to be examined.

It is preferable that the fixing member is a balloon and the balloon is attachably and detachably provided on the insertion part.

It is preferable that the fixing member is a balloon and the balloon is provided integrally with the insertion part.

An endoscope according to another aspect of the present invention comprises an endoscope body, an overtube, and a fixing member. The endoscope body includes an insertion part that is to be inserted into an object to be examined, a distal end part that is provided at a distal end of the insertion part, a bendable part that is connected to a proximal end of the distal end part and changes an orientation of the distal end part by being operated to be bent, an emitting unit that is disposed in the distal end part and emits measurement light, and an image pickup optical system that is provided in the distal end part and picks up an image of an inside of the object to be examined. The overtube covers the insertion part. The fixing member fixes a position of the insertion part with respect to the object to be examined. It is preferable that the fixing member is a balloon and the balloon is provided integrally with the overtube.

It is preferable that the balloon is positioned closer to a proximal end of the insertion part than the bendable part. It is preferable that the measurement light is one or a plurality of spotlights, line-shaped measurement light, grid-pattern measurement light, or three-dimensional plane light.

It is preferable that a subject is intermittently irradiated with the measurement light.

It is preferable that the measurement light is stripe-pattern light. It is preferable that a subject is irradiated with a plurality of types of the stripe-pattern light having different phases or periods while the plurality of types of the stripe-pattern light are switched.

An endoscope system according to still another aspect of the present invention includes an endoscope and an inflation/deflation device. The endoscope comprises an insertion part that is to be inserted into an object to be examined, a distal end part, a bendable part, an emitting unit, an image pickup optical system, and a balloon. The balloon fixes a position of the insertion part with respect to the object to be examined. The inflation/deflation device supplies fluid to the balloon and sucks the fluid from the balloon to inflate or deflate the balloon.

According to the present invention, it is possible to provide an endoscope that can fix the position of an insertion part and allow an object to be observed to be measured with high accuracy.

### BRIEF DESCRIPTION OF THE DRAWINGS

Fig. 1 is a diagram showing the appearance of an endoscope system.
Fig. 2 is a perspective view showing an insertion part and a balloon of an endoscope.
Fig. 3 is a cross-sectional view of main portions of the balloon mounted on the insertion part.
Fig. 4 is a plan view of a distal end part of the endoscope.
Fig. 5 is a block diagram showing the functions of the endoscope system.
Fig. 6 is a graph showing the transmission distribution of a red color filter RF, a green color filter GF, and a blue color filter BF.
Fig. 7 is a block diagram showing the function of a beam light-emitting unit.
Fig. 8 is an image diagram showing a spot SP and a measurement marker.
Fig. 9 is a diagram illustrating a relationship between an optical axis of measurement light and an optical axis of an image pickup optical system.
Fig. 10 is a block diagram showing the functions of a signal processing unit.
Fig. 11 is an image diagram showing a spot and a first measurement marker in a case where an observation distance corresponds to a near end Px.
Fig. 12 is an image diagram showing a spot and a first measurement marker in a case where an observation distance corresponds to an intermediate vicinity Py.
Fig. 13 is an image diagram showing a spot and a first measurement marker in a case where an observation distance corresponds to a far end Pz.
Fig. 14 is an image diagram of an example of a measurement image in which a measurement marker is superimposed on a subject image.
Fig. 15 is an image diagram of an example of a measurement image in which a measurement marker is superimposed on a subject image.
Fig. 16 is an image diagram of an example of a measurement image in which a measurement marker is superimposed on a subject image.
Figs. 17A, 17B, and 17C are diagrams illustrating types of measurement markers, Fig. 12A shows a measurement marker that includes a line segment and gradations on the left side of a spot SP, Fig. 12B shows a measurement marker that includes a line segment and gradations on the lower side of a spot SP, and Fig. 12C shows a measurement marker that includes a line segment and gradations on the upper right side of a spot SP.
Fig. 18 is a diagram illustrating first measurement markers having a cruciform shape, a cruciform shape with gradations, a distorted cruciform shape, a circular-and-cruciform shape, and the shape of a measurement point group.
Fig. 19 is an image diagram showing three concentric circular markers having the same color.
Fig. 20 is an image diagram showing three concentric circular markers having different colors.
Fig. 21 is an image diagram showing distorted concentric circular markers.
Fig. 22 is a diagram illustrating a light emission pattern in which spotlight is intermittently emitted.
Fig. 23 is an image diagram showing an intersection line and gradations.
Fig. 24 is a diagram illustrating a light emission pattern in which line-shaped measurement light is intermittently emitted.
Fig. 25 is a diagram illustrating stripe-pattern light ZPL.
Fig. 26 is a diagram illustrating the light emission patterns of stripe-pattern light ZPL having a phase X, stripe-pattern light ZPL having a phase Y, and stripe-pattern light ZPL having a phase Z.
Fig. 27 is a diagram illustrating grid-pattern measurement light LPL.
Fig. 28 is a diagram illustrating a light emission pattern in which grid-pattern measurement light is intermittently emitted.
Fig. 29 is a diagram illustrating three-dimensional plane light TPL.
Fig. 30 is a diagram illustrating a light emission pattern in which three-dimensional plane light is intermittently emitted.
Fig. 31 shows an example of a state where the insertion part of the endoscope is inserted into an object to be examined and is a diagram illustrating a state where the balloon is kept in a deflated state.
Fig. 32 shows an example of a state where the insertion part of the endoscope is inserted into an object to be examined and is a diagram illustrating a state where the balloon is kept in an inflated state.
Fig. 33 is a diagram showing the appearance of an endoscope system according to a second embodiment.
Fig. 34 is a cross-sectional view of main portions of an overtube and a balloon mounted on an insertion part of the second embodiment.

### DESCRIPTION OF THE PREFERRED EMBODIMENTS

### [First embodiment]

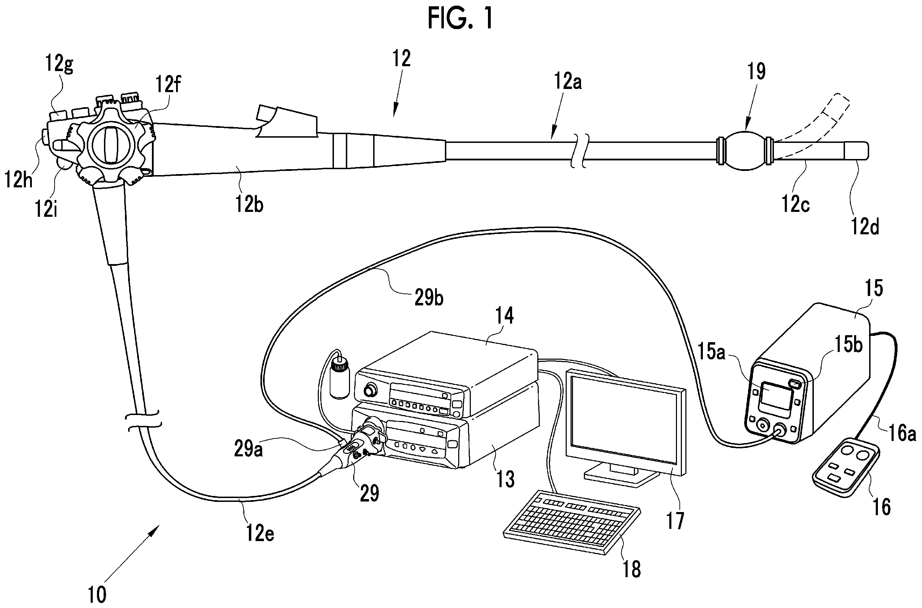

As shown in Fig. 1, an endoscope system 10 includes an endoscope 12, a light source device 13, a processor device 14, a balloon control device 15 (inflation/deflation device), a remote controller 16, a monitor 17, and a user interface 18.

The endoscope 12 includes an insertion part 12a that is to be inserted into an object to be examined, an operation part 12b that is provided at the proximal end portion of the insertion part 12a, a bendable part 12c and a distal end part 12d that are provided on the distal end side of the insertion part 12a, a universal cable 12e, and a balloon 19. An image pickup element, which is used to pick up the image of the inside of the object to be examined, and the like are built in the distal end part 12d. The bendable part 12c is connected to the proximal end of the distal end part 12d, and is operated to be bent in a case where angle knobs 12f of the operation part 12b are operated. The distal end part 12d is made to face in a desired direction by the bending operation of the bendable part 12c.

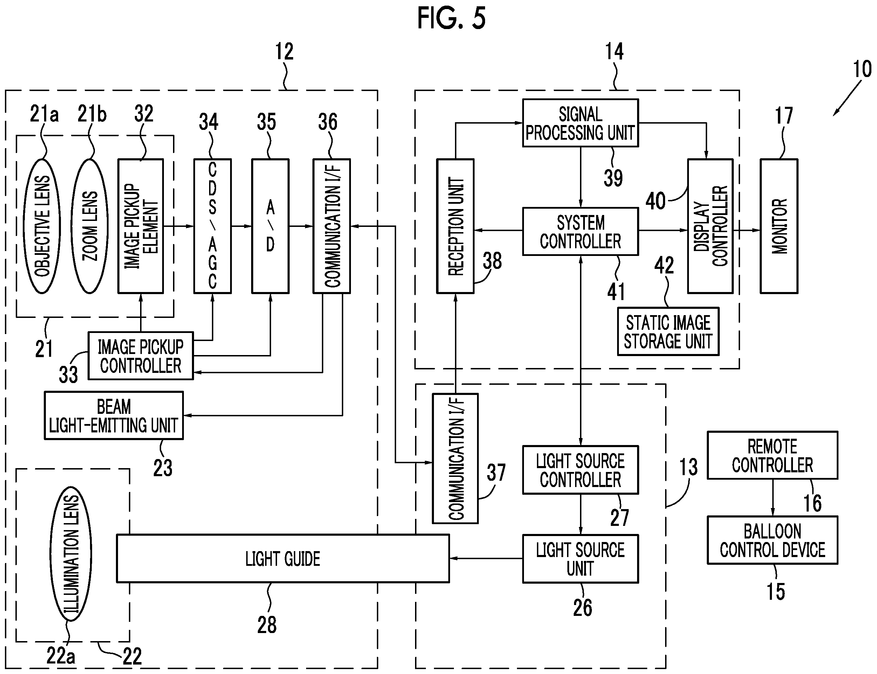

The universal cable 12e is a cable in which a light guide 28 (see Fig. 5) for guiding illumination light for observation emitted from the light source device 13, a control line for transmitting control signals used to control the endoscope 12, a signal line for transmitting image signals obtained from the image pickup of an object to be observed, a power line for supplying power to each part of the endoscope 12, a fluid pipe line for supplying/sucking fluid, and the like are integrated. The distal end of the universal cable 12e is provided with a connector 29 to be connected to the light source device 13.

The light source device 13 generates illumination light using, for example, a semiconductor light source, such as a light emitting diode (LED) or a laser diode (LD), a xenon lamp, a halogen lamp, or the like. In a case where the connector 29 is connected to the light source device 13, illumination light is incident on the light guide 28 (see Fig. 5) of the connector 29 and the object to be observed is irradiated with the illumination light from the distal end of the insertion part 12a.

Further, the light source device 13 is electrically connected to the processor device 14 and the connector 29 of the endoscope 12 is connected to the processor device 14 through the light source device 13. The transmission and reception of control signals, image signals, and the like between the light source device 13 and the connector 29 are performed by wireless communication. For this reason, the light source device 13 transmits the control signals and the like, which are wirelessly transmitted to and received from the connector 29, to the processor device 14. Furthermore, the light source device 13 supplies power, which is used to drive the endoscope 12, to the connector 29, and the supply of this power is also wirelessly performed.

The processor device 14 controls the amount and emission timing of illumination light, which is emitted from the light source device 13, and each part of the endoscope 12 and generates an endoscopic image using image signals that are obtained from the image pickup of the object to be observed irradiated with the illumination light. Further, the processor device 14 is electrically connected to the monitor 17 and the user interface 18. The monitor 17 displays the endoscopic image that is generated by the processor device 14, information about the endoscopic image, and the like. The user interface 18 has a function to receive an input operation, such as function settings.

The endoscope 12 has a normal observation mode, a special light observation mode, and a length measurement mode, and these three modes are switched by a mode changeover switch 12g that is provided on the operation part 12b of the endoscope 12. The normal observation mode is a mode in which an object to be observed is illuminated with the illumination light. The special light observation mode is a mode in which an object to be observed is illuminated with special light different from the illumination light. In the length measurement mode, an object to be observed is illuminated with the illumination light and measurement light and a measurement marker to be used for the measurement of the size and the like of an object to be observed is displayed in a subject image obtained from the image pickup of the object to be observed. The illumination light is light that is used to observe the entire object to be observed by applying brightness to the entire object to be observed. The special light is light that is used to enhance a specific region, such as superficial blood vessels, of the object to be observed. The measurement light is light that is used for the display of the measurement marker.

Further, the operation part 12b of the endoscope 12 is provided with a freeze switch 12h that is used to give a static image-acquisition instruction to acquire the static image of a subject image. In a case where a user operates the freeze switch 12h, the screen of the monitor 17 is frozen and an alert sound (for example, "beep") informing the acquisition of a static image is generated together. Then, the static images of the subject image, which are obtained before and after the operation timing of the freeze switch 12h, are stored in a static image storage unit 42 (see Fig. 5) provided in the processor device 14. The static image storage unit 42 is a storage unit, such as a hard disk, a universal serial bus (USB) memory, or a non-volatile memory. In a case where the processor device 14 can be connected to a network, the static image of the subject image may be stored in a static image storage server (not shown), which is connected to the network, instead of or in addition to the static image storage unit 42. Further, in a case where the static image storage unit 42 is a non-volatile memory, static images may be temporarily stored in the static image storage unit 42 and then transmitted to a USB memory, a compact flash (CF) card, a static image storage server connected to the network, or the like.

A static image-acquisition instruction may be given using an operation device other than the freeze switch 12h. For example, a foot pedal may be connected to the processor device 14, and a static image-acquisition instruction may be given in a case where a user operates the foot pedal (not shown) with a foot. A static image-acquisition instruction may be given by a foot pedal that is used to switch a mode. Further, a gesture recognition unit (not shown), which recognizes the gestures of a user, may be connected to the processor device 14, and may be adapted to give a static image-acquisition instruction in a case where the gesture recognition unit recognizes a specific gesture of a user. The gesture recognition unit may also be used to switch a mode.

Furthermore, a sight line input unit (not shown), which is provided close to the monitor 17, may be connected to the processor device 14, and may be adapted to give a static image-acquisition instruction in a case where the sight line input unit recognizes that a user's sight line is in a predetermined region of the monitor 17 for a predetermined time or longer. Moreover, a voice recognition unit (not shown) may be connected to the processor device 14, and may be adapted to give a static image-acquisition instruction in a case where the voice recognition unit recognizes a specific voice generated by a user. The voice recognition unit may also be used to switch a mode. Moreover, an operation panel (not shown), such as a touch panel, may be connected to the processor device 14, and may be adapted to give a static image-acquisition instruction in a case where a user performs a specific operation on the operation panel. The operation panel may also be used to switch a mode.

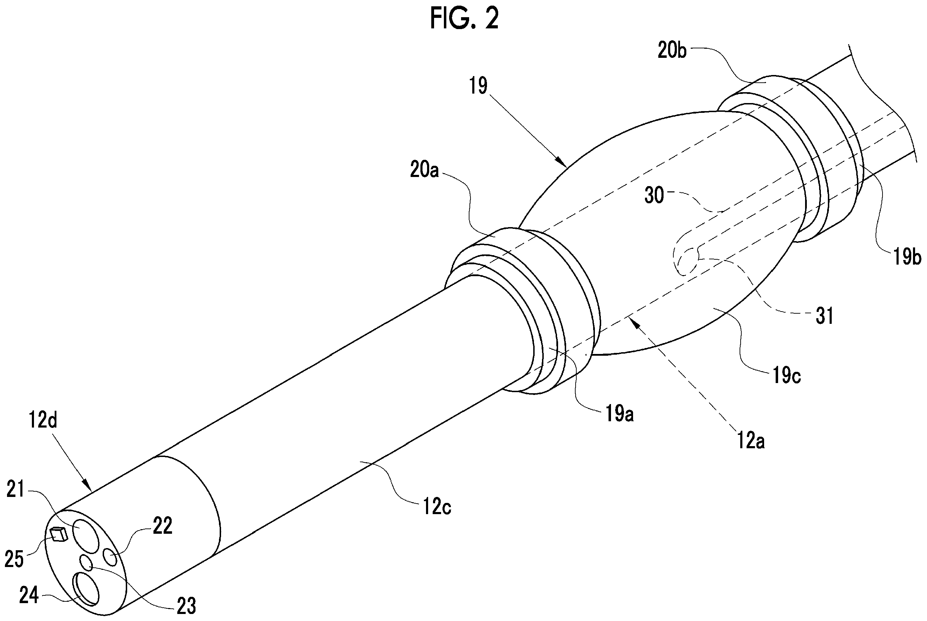

As shown in Fig. 2, a balloon 19 as a fixing member is attachably and detachably mounted on the insertion part 12a. The balloon 19 is a disposable type balloon, is discarded after being used one time or a small number of times, and is replaced with a new one. The number of times mentioned here means the number of cases, and a small number of times refer to 10 times or less.

The balloon 19 is formed in a substantially tubular shape, of which end portions are narrowed, using an elastic material, such as rubber. The balloon 19 includes a distal end portion 19a and a proximal end portion 19b having a small diameter and an intermediate bulging portion 19c. The insertion part 12a is inserted into the balloon 19, the balloon 19 is disposed at a predetermined position, and rings 20a and 20b made of rubber are then fitted to the distal end portion 19a and the proximal end portion 19b, so that the balloon 19 is fixed to the insertion part 12a.

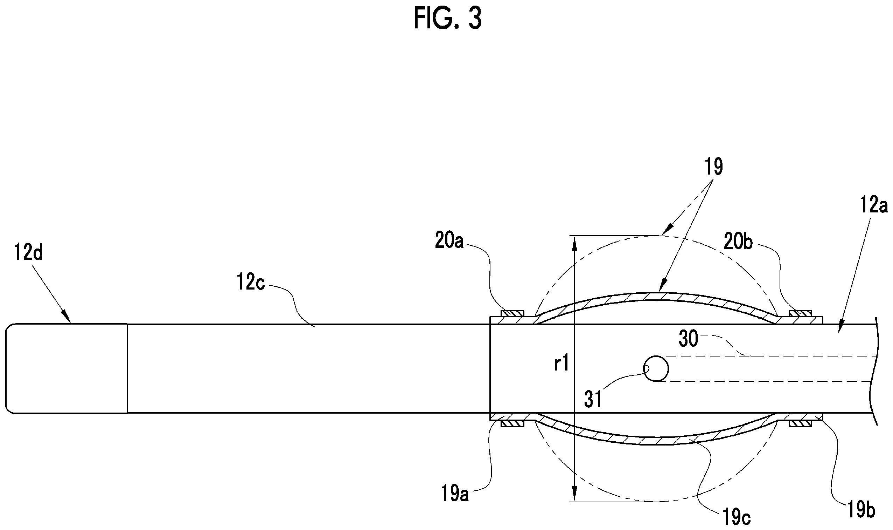

It is preferable that, as shown in Fig. 3, the predetermined position at which the balloon 19 is fixed to the insertion part 12a is closer to the proximal end of the insertion part 12a than the bendable part 12c and is a position where the distal end of the distal end portion 19a of the balloon 19 and the proximal end of the bendable part 12c coincide with each other. Accordingly, the balloon 19 does not hinder the bending operation of the bendable part 12c and the bendable part 12c also does not hinder the inflation and deflation of the balloon 19. As described later, the inflation and deflation of the balloon 19 are controlled by the balloon control device 15.

A specific structure for inflating and deflating the balloon 19 will be described. The endoscope 12 is provided with a fluid pipe line 30 that is used to supply fluid to the balloon 19 and to suck fluid from the balloon 19. The fluid pipe line 30 is disposed in the insertion part 12a, the operation part 12b, the universal cable 12e, and the connector 29.

The fluid pipe line 30 is formed of a tube having flexibility. The fluid pipe line 30 communicates with an opening 31 for the balloon 19 that is formed on the outer peripheral surface of the insertion part 12a. The opening 31 is formed at the mounting position of the balloon 19 and fluid is supplied or sucked from the opening 31, so that the balloon 19 is inflated or deflated. The proximal end of the fluid pipe line 30 is provided with an endoscope-side cap 29a (see Fig. 1).

The endoscope-side cap 29a is formed integrally with the connector 29. A tube 29b is connected to the endoscope-side cap 29a, and the tube 29b is connected to the balloon control device 15. Fluid is supplied or sucked by the balloon control device 15, so that the balloon 19 is inflated or deflated.

The balloon control device 15 is to supply fluid (for example, air) to the balloon 19 or to suck fluid from the balloon 19 in order to inflate or deflate the balloon 19, and is provided with a pump, a solenoid valve, and the like. A remote controller 16 is electrically connected to the balloon control device 15 through the cable 16a.

The balloon control device 15 supplies fluid to the balloon 19 to inflate the balloon 19 or controls the pressure of the fluid to a constant value to keep the balloon 19 in an inflated state (a state shown by a two-dot chain line of Fig. 3). Further, the balloon control device 15 sucks fluid from the balloon 19 to deflate the balloon 19 or controls the pressure of the fluid to a constant value to keep the balloon 19 in a deflated state (a state shown by a solid line of Fig. 3).

In a case where the balloon 19 is inflated, the balloon 19 has, for example, a spherical shape, an elliptical spherical shape, or the shape of a barrel similar thereto. In a case where the endoscope 12 is, for example, a colonofiberscope, the outer diameter r1 of the inflated balloon 19 is formed depending on the inner diameter of the intestinal canal of a large intestine. The balloon 19 is formed so as to have a pressure enough to hold the intestinal canal without perforating the intestinal canal or causing scratches.

A display unit 15a is provided on the front surface of the balloon control device 15. The value of the pressure and the inflated or deflated state of the balloon 19 are displayed on the display unit 15a in a case where the balloon 19 is inflated or deflated. Further, in a case where an abnormality, such as the tear of the balloon 19, occurs, an error code is displayed on the display unit 15a. The value of the pressure and the inflated or deflated state of the balloon 19 may be superimposed on the observation image of the endoscope 12 and displayed on the monitor 17. Furthermore, the balloon control device 15 is provided with a power switch 15b and the like.

Further, the 29b for the supply and suction of fluid to and from the balloon 19 is mounted on the front panel of the balloon control device 15. A back-flow prevention unit (not shown) is provided at a connecting portion between the tube 29b and the balloon control device 15. The back-flow prevention unit has a configuration in which a filter for gas-liquid separation is incorporated into a hollow disk-shaped case attachably and detachably mounted on the front panel of the balloon control device 15; and prevents liquid, such as body fluid, from flowing into the balloon control device 15 in a case where the balloon 19 is torn.

The remote controller 16 is provided with various switches. For example, the remote controller 16 is provided with a stop switch to be operated in a case where an abnormality occurs or the like, an instruction switch to be used to inflate and deflate the balloon 19, and the like.

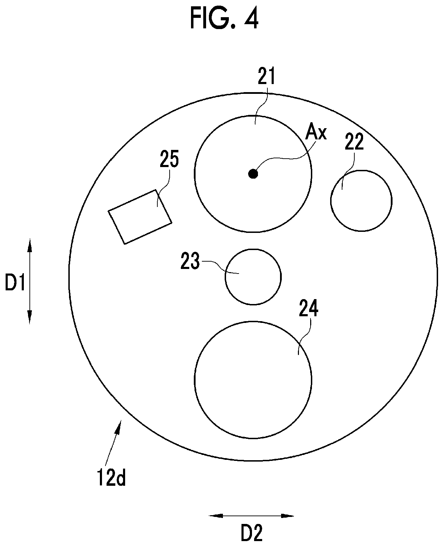

As shown in Fig. 4, the distal end part 12d of the endoscope 12 has a substantially circular shape, and is provided with an image pickup optical system 21 that receives light from a subject, an illumination optical system 22 that is used to irradiate the subject with the illumination light, a beam light-emitting unit 23 that emits the measurement light to the subject, an opening 24 that allows a treatment tool to protrude toward the subject, and an air/water supply nozzle 25 that is used to supply air and water. The beam light-emitting unit 23 corresponds to an emitting unit of claims.

An optical axis Ax of the image pickup optical system 21 extends in a direction perpendicular to the plane of paper. A vertical first direction D1 is orthogonal to the optical axis Ax, and a horizontal second direction D2 is orthogonal to the optical axis Ax and the first direction D1. The image pickup optical system 21 and the beam light-emitting unit 23 are provided at different positions in the distal end part 12d, and are arranged in the first direction D1.

As shown in Fig. 5, the light source device 13 comprises a light source unit 26 and a light source controller 27. The light source unit 26 generates the illumination light or the special light that is used to illuminate the subject. The illumination light or the special light, which is emitted from the light source unit 26, is incident on the light guide 28, and the subject is irradiated with the illumination light or the special light through an illumination lens 22a. A white light source emitting white light, a plurality of light sources, which include a white light source and a light source emitting another color light (for example, a blue light source emitting blue light), or the like is used as a light source of the illumination light in the light source unit 26. Further, a light source, which emits broadband light including blue narrow-band light used to emphasize superficial information about superficial blood vessels and the like, is used as a light source of the special light in the light source unit 26. The light source controller 27 is connected to a system controller 41 of the processor device 14. Mixed white light, which is a combination of blue light, green light, and red light, may be used as the illumination light. In this case, it is preferable that the illumination optical system 22 is optically designed to allow the irradiation range of green light to be wider than the irradiation range of red light.

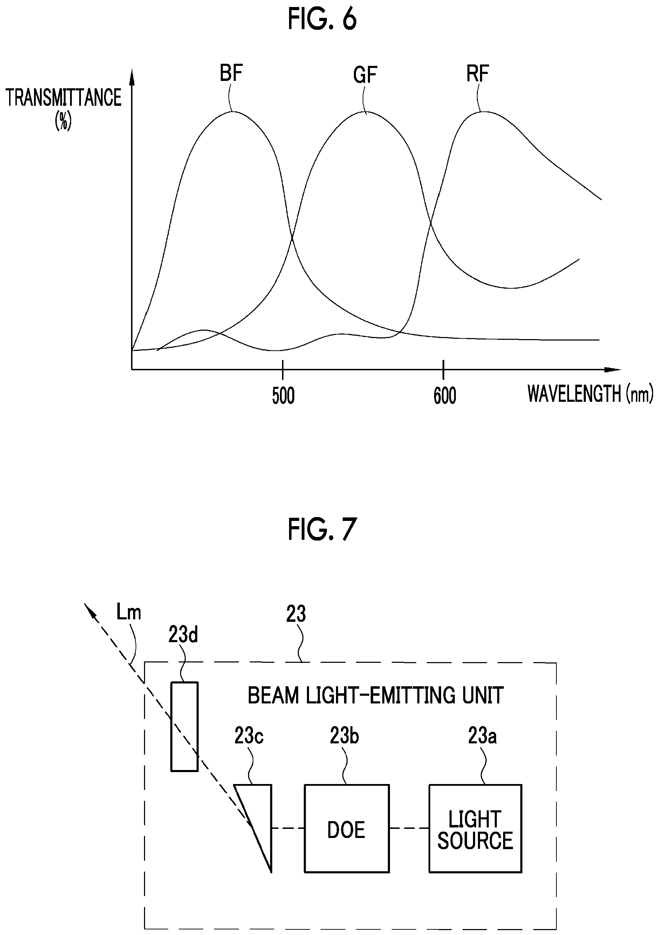

The light source controller 27 controls the light source unit 26 on the basis of an instruction given from the system controller 41. The system controller 41 not only gives an instruction related to light source control to the light source controller 27 but also controls a light source 23a (see Fig. 7) of the beam light-emitting unit 23. In the case of the normal observation mode, the system controller 41 performs a control to turn on the illumination light and to turn off the measurement light. In the case of the special light observation mode, the system controller 41 performs a control to turn on the special light and to turn off the measurement light. In the case of the length measurement mode, the system controller 41 performs a control to turn on the illumination light and to turn on the measurement light.

The illumination optical system 22 includes the illumination lens 22a, and the object to be observed is irradiated with light, which is emitted from the light guide 28, through the illumination lens 22a. The image pickup optical system 21 includes an objective lens 21a, a zoom lens 21b, and an image pickup element 32. Light reflected from the object to be observed is incident on the image pickup element 32 through the objective lens 21a and the zoom lens 21b. Accordingly, the reflected image of the object to be observed is formed on the image pickup element 32.

The zoom lens 21b has an optical zoom function to enlarge or reduce the subject as a zoom function by moving between a telephoto end and a wide end. ON/OFF of the optical zoom function can be switched by a zoom operation part 12i (see Fig. 1) provided on the operation part 12b of the endoscope, and the subject can be enlarged or reduced at a specific magnification ratio in a case where the zoom operation part 12i is further operated in a state where the optical zoom function is ON.

The image pickup element 32 is a color image pickup sensor, and picks up the reflected image of an object to be examined and outputs image signals. It is preferable that the image pickup element 32 is a charge coupled device (CCD) image pickup sensor, a complementary metal-oxide semiconductor (CMOS) image pickup sensor, or the like. The image pickup element 32 used in the embodiment of the present invention is a color image pickup sensor that is used to obtain red images, green images, and red images corresponding to three colors of R (red), G (green), and B (blue). The red image is an image that is output from red pixels provided with red color filters in the image pickup element 32. The green image is an image that is output from green pixels provided with green color filters in the image pickup element 32. The blue image is an image that is output from blue pixels provided with blue color filters in the image pickup element 32. The image pickup element 32 is controlled by an image pickup controller 33.

Since a red color filter RF has high transmittance in a red-light wavelength range of 600 to 700 nm as shown in Fig. 6, a red pixel has high sensitivity in the red-light wavelength range. Since a green color filter GF has high transmittance in a green-light wavelength range of 500 to 600 nm, a green pixel has high sensitivity in the green-light wavelength range. Since a blue color filter BF has high transmittance in a blue-light wavelength range of 400 to 500 nm, a blue pixel has high sensitivity in the blue-light wavelength range. A red pixel has sensitivity even in the green-light wavelength range or the blue-light wavelength range. Further, a green pixel has sensitivity even in the red-light wavelength range or the blue-light wavelength range. Furthermore, a blue pixel has sensitivity even in the red-light wavelength range or the green-light wavelength range.

Image signals output from the image pickup element 32 are transmitted to a CDS/AGC circuit 34. The CDS/AGC circuit 34 performs correlated double sampling (CDS) or auto gain control (AGC) on the image signals that are analog signals. The image signals, which have been transmitted through the CDS/AGC circuit 34, are converted into digital image signals by an analog/digital converter (A/D converter) 35. The digital image signals, which have been subjected to A/D conversion, are input to a communication interface (I/F) 37 of the light source device 13 through a communication interface (I/F) 36.

The processor device 14 comprises a reception unit 38 that is connected to the communication Interface (I/F) 37 of the light source device 13, a signal processing unit 39, a display controller 40, and the system controller 41. The communication I/F receives the image signals, which are transmitted from the communication I/F 37, and transmits the image signals to the signal processing unit 39. A memory, which temporarily stores the image signals received from the reception unit 38, is built in the signal processing unit 39, and the signal processing unit 39 processes an image signal group, which is a set of the image signals stored in the memory, to generate the subject image. The reception unit 38 may directly transmit control signals, which are related to the light source controller 27, to the system controller 41. Further, a processing unit or a storage unit (for example, an irradiation region-recognizing section 58 or a marker table 62), which are related to the length measurement mode, of the processor device 14 may be provided in a length measurement processor (not shown) separate from the processor device 14. In this case, the length measurement processor and the processor device 14 are in a state where the length measurement processor and the processor device 14 can communicate with each other so that images or various types of information can be transmitted and received.

In a case where the endoscope 12 is set to the normal observation mode, signal assignment processing for assigning the blue image of the subject image to B channels of the monitor 17, assigning the green image of the subject image to G channels of the monitor 17, and assigning the red image of the subject image to R channels of the monitor 17 is performed in the signal processing unit 39. As a result, a color subject image is displayed on the monitor 17. The same signal assignment processing as that in the normal observation mode is performed even in the length measurement mode. On the other hand, in a case where the endoscope 12 is set to the special light observation mode, the red image of the subject image is not used for the display of the monitor 17, the blue image of the subject image is assigned to the B channels and the G channels of the monitor 17, and the green image of the subject image is assigned to the R channels of the monitor 17 in the signal processing unit 39. As a result, a pseudo color subject image is displayed on the monitor 17. In a case where the endoscope 12 is set to the length measurement mode, the signal processing unit 39 may be adapted to perform structure enhancement processing for enhancing structures, such as blood vessels, or color difference enhancement processing for increasing a color difference between a normal area and a lesion area of the object to be observed on the subject image.

The display controller 40 causes the monitor 17 to display the subject image that is generated by the signal processing unit 39. The system controller 41 performs the control of the image pickup element 32 through the image pickup controller 33 provided in the endoscope 12. The image pickup controller 33 also performs the control of the CDS/AGC circuit 34 and the A/D converter 35 in accordance with the control of the image pickup element 32.

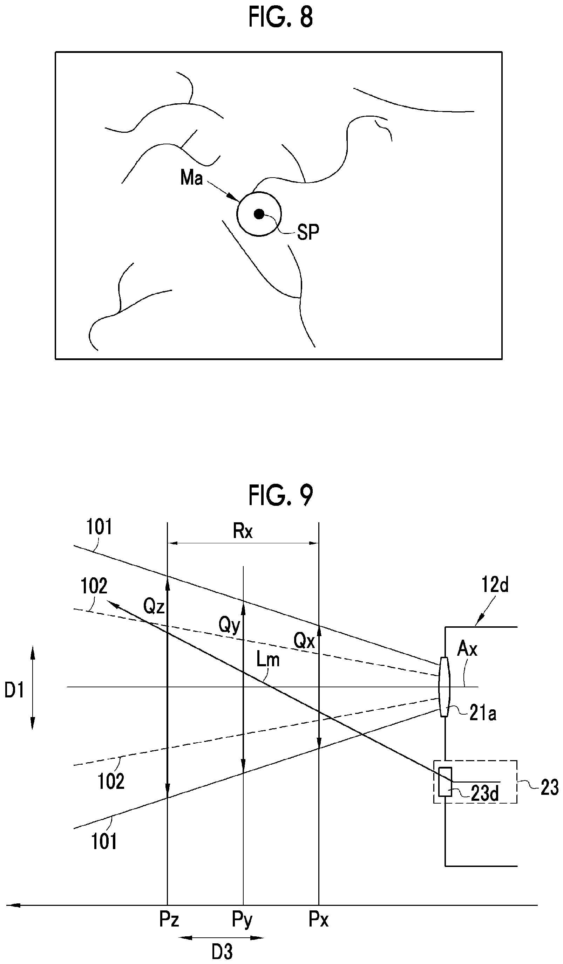

As shown in Fig. 7, the beam light-emitting unit 23 emits measurement light that is inclined with respect to the optical axis Ax (see Fig. 9) of the image pickup optical system 21. The beam light-emitting unit 23 comprises a light source 23a, a diffractive optical element (DOE) 23b, a prism 23c, and an emitting section 23d. The light source 23a is to emit light having a color that can be detected by pixels of the image pickup element 32 (specifically visible light), and includes a light emitting element, such as a laser light source (laser diode (LD)) or a light emitting diode (LED), and a condenser lens that condenses light emitted from the light emitting element. The light source 23a is provided on a scope electric board (not shown). The scope electric board is provided in the distal end part 12d of the endoscope, and receives power supplied from the light source device 13 or the processor device 14 and supplies power to the light source 23a.

For example, red (the color of beam light) laser light having a wavelength of 600 nm or more and 650 nm or less is used as the light emitted from the light source 23a in this embodiment, but light having a wavelength in other ranges, for example, green light having a wavelength of 495 nm or more and 570 nm or less or blue light may be used. The light source 23a is controlled by the system controller 41, and emits light on the basis of an instruction given from the system controller 41. The DOE 23b converts the light, which is emitted from the light source, into the measurement light that is used to obtain measurement information. It is preferable that the amount of measurement light is adjusted from a standpoint of protecting a human body, eyes, and internal organs and is adjusted to the amount of light enough to cause halation (pixel saturation) in the observation range of the endoscope 12.

The prism 23c is an optical member that is used to change the travel direction of the measurement light converted by the DOE 23b. The prism 23c changes the travel direction of the measurement light so that the measurement light intersects with the visual field of the image pickup optical system 21 including the objective lens 21a. The details of the travel direction of the measurement light will also be described later. A subject is irradiated with measurement light, which is emitted from the prism 23c, through the emitting section 23d that is formed of an optical member.

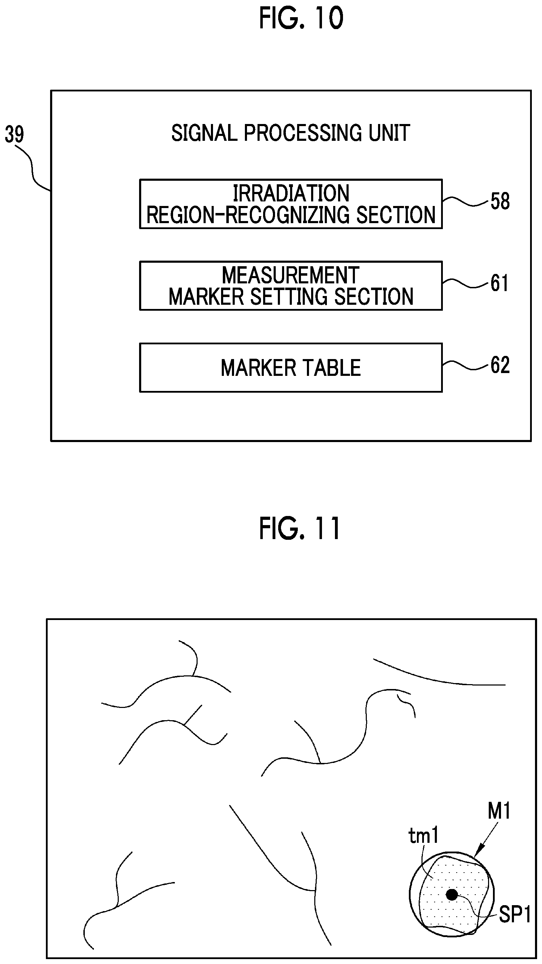

In a case where the subject is irradiated with the measurement light, a spot SP as an irradiation region is formed on the subject as shown in Fig. 8. The position of the spot SP is recognized by the irradiation region-recognizing section 58 (see Fig. 10), and a measurement marker showing the size of the subject is set according to the position of the spot SP. The display controller 40 causes the monitor 17 to display a measurement image in which the set measurement marker is displayed in the subject image.

The position of the spot SP and an observation distance (a distance between the distal end part 12d of the endoscope and the object to be observed) are related to each other. The observation distance is shorter as the position of the spot SP is closer to the lower side, and the observation distance is longer as the position of the spot SP is closer to the upper side. Accordingly, it is possible to measure an observation distance according to the position of the spot SP. A set measurement marker Ma is displayed in the subject image. The radius of the measurement marker Ma from the spot SP shows the actual size (for example, 5 mm) of the subject. Further, the size (radius) of the measurement marker Ma changes depending on the position of the spot SP, that is, the observation distance. For example, the size of the measurement marker Ma is larger as the position of the spot SP is closer to the lower side, and the size of the measurement marker Ma is smaller as the position of the spot SP is closer to the upper side.

A plurality of types of measurement markers, such as a first measurement marker and a second measurement marker, are included in the measurement marker as described later, and a measurement marker to be displayed in the subject image among the plurality of types of measurement markers can be selected according to a user's instruction. For example, the user interface 18 is used for the user's instruction.

The emitting section 23d may be formed of an auxiliary measurement slit, which is formed at the distal end part 12d of the endoscope, instead of being formed of an optical member. Further, in a case where the emitting section 23d is formed of an optical member, it is preferable that an anti-reflection coating (AR coating) (anti-reflection portion) is provided on the emitting section 23d. The reason why the anti-reflection coating is provided as described above is that it is difficult for the irradiation region-recognizing section 58 to be described later to recognize the position of the spot SP formed on the subject by the measurement light in a case where the measurement light is reflected without being transmitted through the emitting section 23d and a ratio of the measurement light with which a subject is irradiated is reduced.

The beam light-emitting unit 23 has only to be capable of emitting the measurement light to the visual field of the image pickup optical system 21. For example, the light source 23a may be provided in the light source device and light emitted from the light source 23a may be guided to the DOE 23b by optical fibers. Further, the prism 23c may not be used and the orientations of the light source 23a and the DOE 23b may be inclined with respect to the optical axis Ax of the image pickup optical system 21 so that measurement light is emitted in a direction crossing the visual field of the image pickup optical system 21.

With regard to the travel direction of the measurement light, the measurement light is emitted in a state where the optical axis Lm of the measurement light intersects with the optical axis Ax of the image pickup optical system 21 as shown in Fig. 9. In a case where the subject can be observed in a range Rx of an observation distance, it is understood that the positions (points where the respective arrows Qx, Qy, and Qz intersect with the optical axis Ax) of the spot SP, which is formed on the subject by the measurement light, in image pickup ranges (shown by arrows Qx, Qy, and Qz) at a near end Px, an intermediate vicinity Py, and a far end Pz of the range Rx are different from each other. The image pickup angle of view of the image pickup optical system 21 is represented by a region between two solid lines 101, and measurement is performed in a central region (a region between two dotted lines 102), in which an aberration is small, of this image pickup angle of view.

Since the measurement light is emitted in a state where the optical axis Lm of the measurement light intersects with the optical axis Ax as described above, the size of the subject can be measured from the movement of the position of the spot with respect to a change in observation distance. Then, the image of the subject illuminated with the measurement light is picked up by the image pickup element 32, so that a subject image including the spot SP is obtained. In the subject image, the position of the spot SP varies depending on a relationship between the optical axis Ax of the image pickup optical system 21 and the optical axis Lm of the measurement light and an observation distance. However, the number of pixels showing the same actual size (for example, 5 mm) is increased in the case of a short observation distance, and the number of pixels showing the same actual size is reduced in the case of a long observation distance.

As shown in Fig. 10, the signal processing unit 39 of the processor device 14 comprises an irradiation region-recognizing section 58, a measurement marker setting section 61, and a marker table 62. In a case where the endoscope 12 is set to the normal observation mode, the subject image of the subject illuminated with the illumination light is input to the signal processing unit 39. In a case where the endoscope 12 is set to the special light observation mode, the subject image of the subject illuminated with the special light is input to the signal processing unit 39. In a case where the endoscope 12 is set to the length measurement mode, the subject image of the subject illuminated with the illumination light and the measurement beam light is input to the signal processing unit 39.

The irradiation region-recognizing section 58 recognizes a beam irradiation region, which has a pattern having a specific shape, from the subject image. In a case where the beam irradiation region is the above-mentioned spot SP, the pattern having a specific shape has a substantially circular shape as shown in Fig. 8.

The measurement marker setting section 61 sets a first measurement marker, which shows the actual size (the same size as the actual size) of the subject, as a measurement marker, which is used to measure the size of the subject, on the basis of the position of the spot SP. The measurement marker setting section 61 sets a measurement marker image, which corresponds to the position of the spot SP, with reference to the marker table 62 in which a measurement marker image of which the display aspect varies depending on the irradiation position of the spot SP is stored in association with the position of the spot SP. In this case, with regard to a positional relationship between the measurement marker and the spot SP, the spot SP is positioned at any one of, for example, the "centroid", "center", or "coordinates regarded as the center" of the measurement marker.

For example, the size or shape of the measurement marker image varies depending on the irradiation position of the spot SP. Further, the contents stored in the marker table 62 are maintained even in a case where the power of the processor device 14 is turned off. The measurement marker image and the irradiation position are stored in the marker table 62 in association with each other, but a distance to the subject (a distance between the distal end part 12d of the endoscope 12 and the subject) corresponding to the irradiation position and the measurement marker image may be stored in the marker table 62 in association with each other.

The display controller 40 causes the monitor 17 to display the measurement image in which a measurement marker is displayed in the subject image on the basis of the position of the spot SP that is an irradiation region. Specifically, the display controller 40 causes the monitor 17 to display the measurement image in which the first measurement marker is superimposed to center on the spot SP. For example, a circular measurement marker is used as the first measurement marker. In this case, as shown in Fig. 11, a marker M1, which shows an actual size of 5 mm (a horizontal direction and a vertical direction of the subject image), is displayed at the center of a spot SP1 formed on a tumor tm1 of a subject in a case where an observation distance is close to the near end Px. In a case where the measurement marker is displayed on the monitor 17, an observation distance may also be displayed on the monitor 17 together.

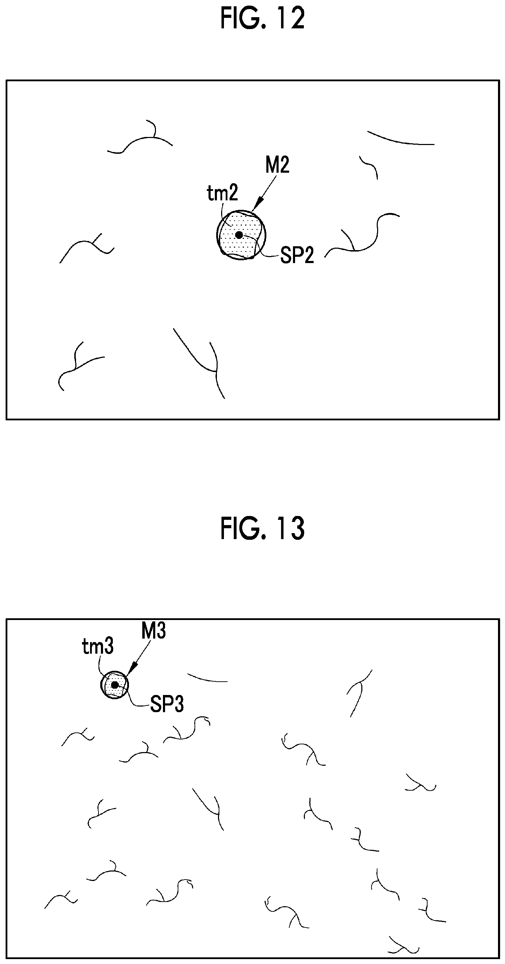

Further, as shown in Fig. 12, a marker M2, which shows an actual size of 5 mm (the horizontal direction and the vertical direction of the subject image), is displayed at the center of a spot SP2 formed on a tumor tm2 of a subject in a case where an observation distance is close to the intermediate vicinity Py. Since the marker display position of the marker M2 is positioned at the central portion of the subject image that is less likely to be affected by distortion caused by the image pickup optical system 21, the marker M2 has a circular shape without being affected by the distortion or the like.

Furthermore, as shown in Fig. 13, a marker M3, which shows an actual size of 5 mm (the horizontal direction and the vertical direction of the subject image), is displayed at the center of a spot SP3 formed on a tumor tm3 of a subject. As shown in Figs. 11 to 13 having been described above, the size of the first measurement marker corresponding to the same actual size of 5 mm is reduced with an increase in an observation distance. Moreover, the shape of the first measurement marker also varies depending on the marker display position due to an influence of the distortion caused by the image pickup optical system 21.

In Figs. 11 to 13, the center of the spot SP and the center of the marker are displayed to coincide with each other. However, the first measurement marker may be displayed at a position away from the spot SP in a case where there is no problem in measurement accuracy. Even in this case, it is preferable that the first measurement marker is displayed near the spot.

The first measurement marker corresponding to the actual size of the subject, which is 5 mm, is displayed in Figs. 11 to 13, but the actual size of the subject may be set to any value (for example, 2 mm, 3 mm, 10 mm, or the like) according to an object to be observed or the purpose of observation.

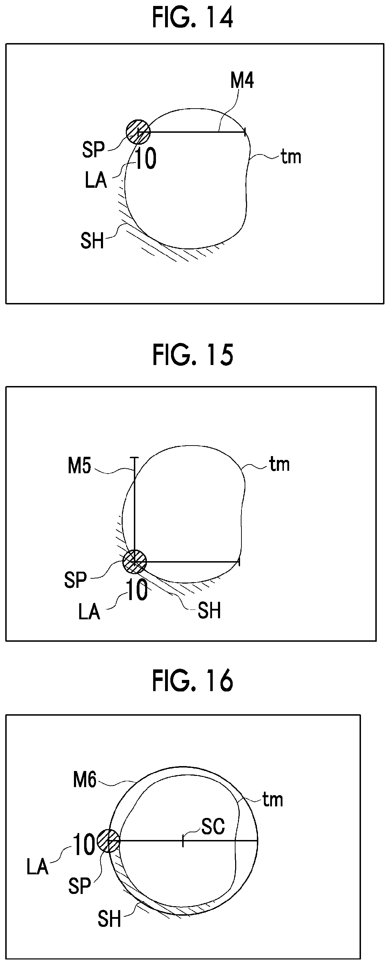

A positional relationship between the measurement marker and the spot SP is not limited to a relationship in which the spot SP is positioned at any one of the "centroid", "center", or "coordinates regarded as the center" of the measurement marker as shown in Figs. 11 to 13, and the shape of the measurement marker is not limited to a circular shape. For example, as shown in Figs. 14 to 17, the measurement marker setting section 61 may set a measurement marker that corresponds to the position of the spot SP and includes gradations of which an end portion serves as a base point. The end portion is a portion closer to the outer portion than a central portion, or a starting point, an end point, or the like in the shape of the measurement marker.

Fig. 14 shows a measurement image in which a marker M4 set by the measurement marker setting section 61 is superimposed on a subject image so that the position of the spot SP and the base point of the gradations of the marker M4 overlap with each other. Since a tumor tm has a three-dimensional shape in Figs. 14 to 17, the subject image includes the tumor tm and the spot SP and includes a shadow SH in some cases. It is preferable that the marker M4 is superimposed to be displayed at the position of the spot SP for more accurate measurement. Accordingly, it is preferable that the marker M4 is displayed close to the spot SP as much as possible even in a case where the marker M4 is displayed at a position away from the spot SP. The marker M4 is a straight line segment, and includes gradations, which are line segments perpendicular to the straight line segment, at the starting point and the end point of the line segment. In a case where the marker M4 is a line segment or the like and has a starting point and an end point, the starting point and/or the end point themselves may be used as gradations. In this case, for example, gradations having the shape of a line segment perpendicular to the straight line segment may not be provided.

Further, the marker M4 may include a numeral "10" near the base point of the gradations. This is a gradation label LA of the marker M4, and is given so that the line segment of the marker M4 can be easily recognized as an actual size of 10 mm. Hereinafter, numerals included in measurement markers have the same meaning. The numerical value of the gradation label LA can be changed by setting, and a marker M4 of which a gradation label LA itself is not displayed may be provided.

Various types of measurement markers are used depending on settings. For example, a measurement marker having the shape of a straight line segment or a shape in which straight line segments are combined with each other, a measurement marker having a circular shape or a shape in which circles are combined with each other, or a measurement marker having a shape in which a circle and a straight line segment are combined with each other, and the like are used.

For example, a measurement image shown in Fig. 15 includes a marker M5 having a shape in which straight line segments are combined with each other. The marker M5 has a shape in which straight line segments are combined in an L shape, the line segments extend upward along the plane of paper and in a direction along the plane of paper from the corner of the L shape as a base point, and the marker M5 includes a gradation at each of end points of the line segments with the base point as a starting point. Further, the marker M5 includes a numeral "10", which is a gradation label LA, near the base point of the gradations like the marker M4.

For example, a measurement image shown in Fig. 16 includes a marker M6 having a shape in which a straight line segment and a circle are combined with each other. The marker M6 has a shape in which a circle and a line segment corresponding to the diameter of the circle are combined with each other, and the line segment extends to the right along the plane of paper from one of intersections between the line segment and the circle as a base point. With regard to the line segment or the circle, the respective intersections between the line segment and the circle are used as gradations. The marker M6 may include a gradation SC at a point where the line segment is divided in half or the center of the circle. Further, the marker M6 includes a numeral "10", which is a gradation label LA, near the base point of the gradations like the marker M4 or the marker M5.

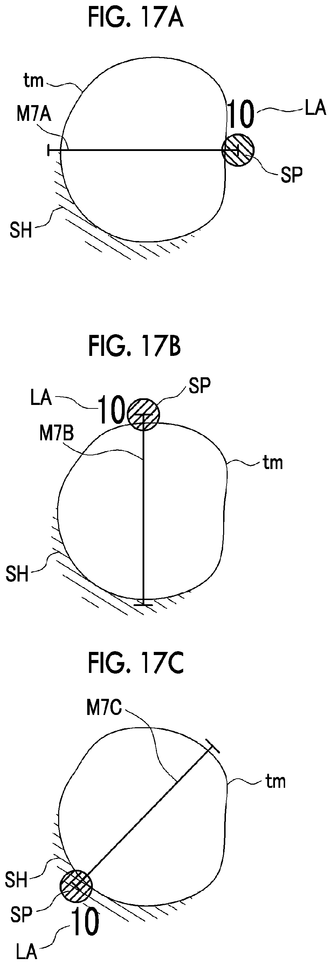

As shown in Figs. 17A, 17B, and 17C, in addition to these, the measurement marker may have various shapes like, for example, a marker M7A (Fig. 17A) of which a line segment extends from a base point to the left along the plane of paper and which includes a gradation label LA, a marker M7B (Fig. 17B) of which a line segment extends downward along the plane of paper from a base point and which includes a gradation label LA, a marker M7C (Fig. 17C) of which a line segment extends in a direction oblique to the upper right side along the plane of paper from a base point and which includes a gradation label LA, or the like.

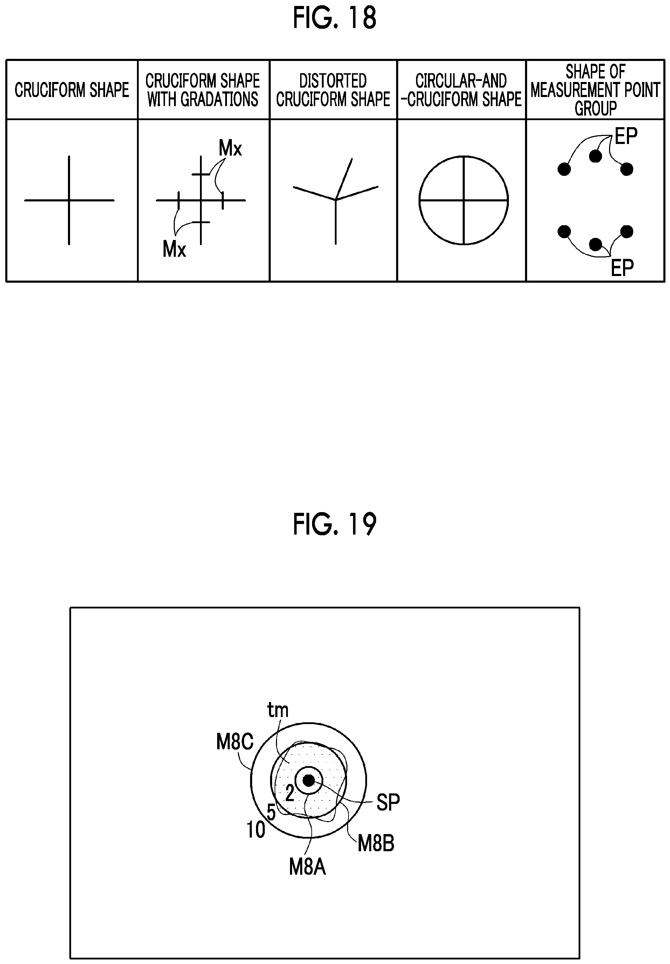

Further, the first measurement marker may have a cruciform shape where a vertical line and a horizontal line intersect with each other as shown in Fig. 18 in addition to these shapes. Furthermore, the first measurement marker may have a cruciform shape with gradations where gradations Mx are given to at least one of a vertical line or a horizontal line of a cruciform shape. Further, the first measurement marker may have a distorted cruciform shape of which at least one of a vertical line or a horizontal line is inclined. Furthermore, the first measurement marker may have a circular-and-cruciform shape where a cruciform shape and a circle are combined with each other. In addition, the first measurement marker may have the shape of a measurement point group where a plurality of measurement points EP corresponding to an actual size from a spot are combined with each other. Further, one first measurement marker may be displayed or a plurality of first measurement markers may be displayed, and the color of the first measurement marker may be changed according to an actual size.

As shown in Fig. 19, three concentric circular markers M8A, M8B, and M8C having different sizes (diameters as the sizes are 2 mm, 5 mm, and 10 mm, respectively) may be displayed in the subject image as the first measurement marker to center on a spot SP formed on a tumor tm. Since the three concentric circular markers are displayed as a plurality of markers, time and effort required to switch a marker can be saved and measurement can be performed even in a case where a subject has a non-linear shape. In a case where a plurality of concentric circular markers are to be displayed to center on a spot, a size and a color are not designated for each marker and combinations of a plurality of conditions may be prepared in advance and one can be selected from these combinations.

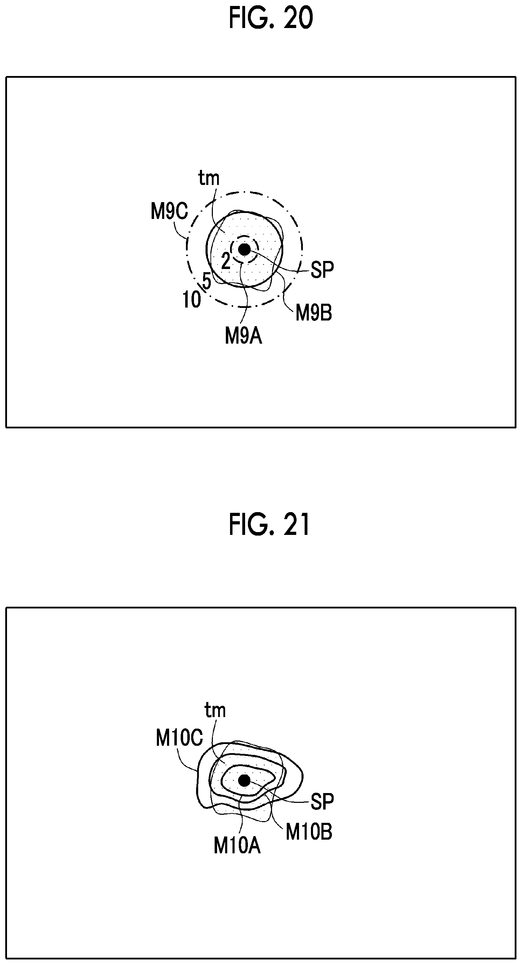

In Fig. 19, all the three concentric circular markers are displayed with the same color (black). However, in a case where a plurality of concentric circular markers are to be displayed, a plurality of color concentric circular markers of which colors are different from each other may be used. As shown in Fig. 20, a marker M9A is displayed by a dotted line representing a red color, a marker M9B is displayed by a solid line representing a blue color, and a marker M9C is displayed by a one-dot chain line representing a white color. Since identifiability can be improved in a case where the colors of the markers are different from each other in this way, measurement can be easily performed.

Further, as shown in Fig. 21, a plurality of distorted concentric circular markers, which are distorted from the respective concentric circles, may be used as the first measurement marker other than the plurality of concentric circular markers. In this case, distorted concentric circular markers M10A, M10B, and M10C are displayed in the subject image to center on a spot SP formed on a tumor tm.

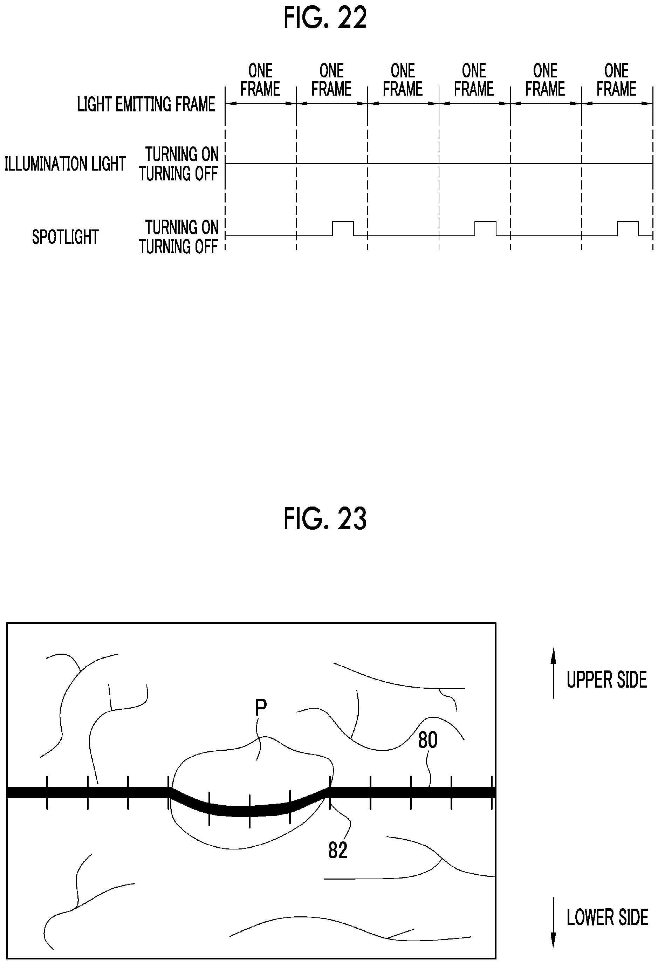

The subject is constantly irradiated with the illumination light and spotlight (measurement light) in the length measurement mode. However, as shown in Fig. 22, the illumination light may be constantly turned on so that the subject is irradiated with the illumination light and the spotlight may be repeatedly turned on and turned off (dimmed) every frame (or every few frames) so that the subject is intermittently irradiated with the spotlight. In this case, in a frame where the spotlight is turned on, the position of the spotlight is detected and the display of a measurement marker is set. Further, it is preferable that the measurement marker of which the display is set is superimposed and displayed on an image obtained in a frame where the subject is irradiated with only the illumination light.

Light, which forms a spot in a case where a subject is irradiated with the light, is used as the measurement light, but other light may be used. For example, line-shaped measurement light, which is formed on the subject as an intersection line 80 as shown in Fig. 23 in a case where the subject is irradiated with the light, may be used. In a case where the subject is irradiated with the line-shaped measurement light, the intersection line 80 as a line-shaped irradiation region is formed on the subject. In this case, a second measurement marker that consists of the intersection line 80 and gradations 82 formed on the intersection line 80 and serving as an index of the size of the subject (for example, a polyp P) is generated as a measurement marker.

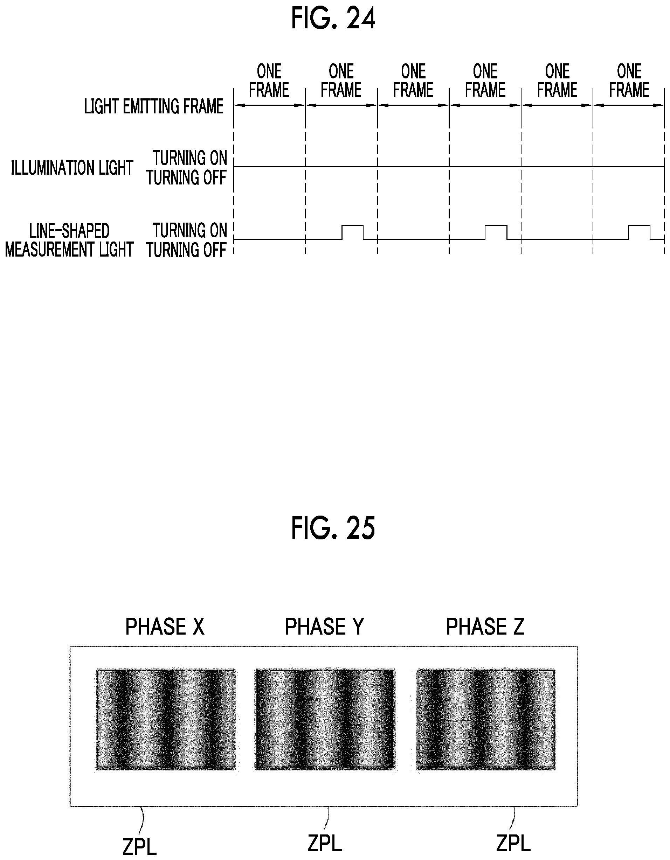

In a case where the line-shaped measurement light is used as the measurement light, in the length measurement mode, the subject may be constantly irradiated with the illumination light and the line-shaped measurement light or the subject may be constantly irradiated with the illumination light and the line-shaped measurement light may be repeatedly turned on and turned off (dimmed) every frame (or every few frames) as shown in Fig. 24 so that the subject is intermittently irradiated with the line-shaped measurement light. In this case, in a frame where the line-shaped measurement light is turned on, the position of the line-shaped measurement light is detected and the display of a measurement marker is set. Further, it is preferable that the measurement marker of which the display is set is superimposed and displayed on an image obtained in a frame where the subject is irradiated with only the illumination light.

Stripe-pattern light ZPL, which is formed as light having a stripe pattern on a subject as shown in Fig. 25 in a case where the subject is irradiated with the light, may be used as the measurement light (for example, see JP2016-198304A). The stripe-pattern light ZPL is obtained in a case where a variable-transmittance liquid crystal shutter (not shown) is irradiated with specific laser light, and is formed of two different vertical stripe patterns in which a region (transmissive region) through which specific laser light is transmitted by the liquid crystal shutter and (non-transmissive region) through which the specific laser light is not transmitted are periodically repeated in the horizontal direction. In a case where the stripe-pattern light is used as the measurement light, the period of the stripe-pattern light changes depending on a distance from the subject. Accordingly, the subject is irradiated with the stripe-pattern light plural times while the period or phase of the stripe-pattern light is shifted by the liquid crystal shutter, and the three-dimensional shape of the subject is measured on the basis of a plurality of images obtained by shifting the period or phase.

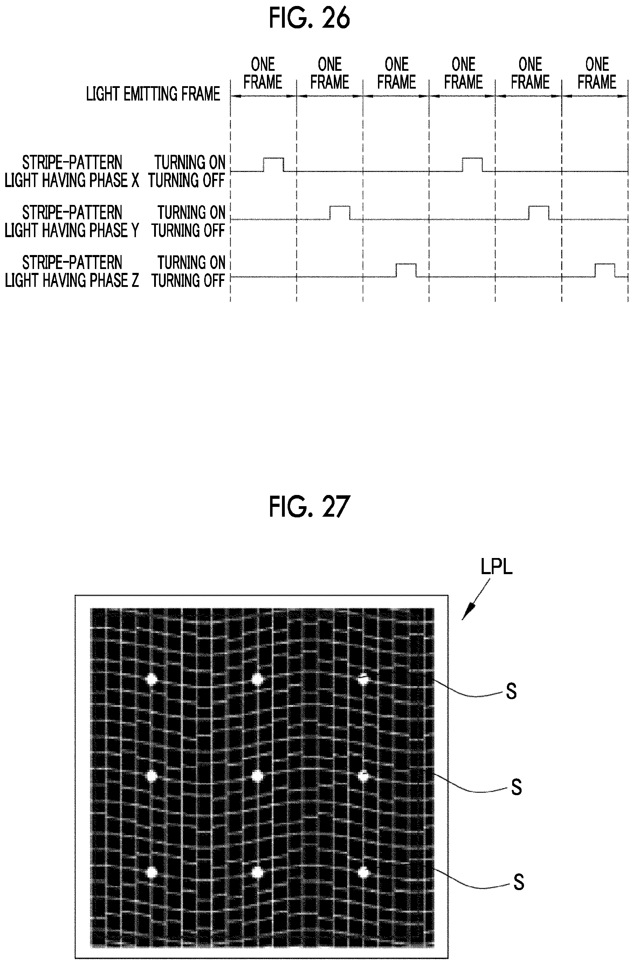

For example, a subject is alternately irradiated with stripe-pattern light having a phase X, stripe-pattern light having a phase Y, and stripe-pattern light having a phase Z. The phases of the vertical stripe patterns of the stripe-pattern light having the phase X, the stripe-pattern light having the phase Y, and stripe-pattern light having the phase Z are shifted from each other by 120° (2π/3). In this case, the three-dimensional shape of the subject is measured using three types of images obtained on the basis of the respective types of stripe-pattern light. For example, it is preferable that the subject is irradiated with the stripe-pattern light having the phase X, the stripe-pattern light having the phase Y, and the stripe-pattern light having the phase Z while the stripe-pattern light having the phase X, the stripe-pattern light having the phase Y, and the stripe-pattern light having the phase Z are switched every frame (or every few frames) as shown in Fig. 26. It is preferable that the subject is constantly irradiated with the illumination light.

Grid-pattern measurement light LPL, which is formed as a grid pattern as shown in Fig. 27 in a case where a subject is irradiated with the light, may be used as the measurement light (for example, see JP2017-217215A). In this case, since the three-dimensional shape of the subject is measured according to the state of deformation of the grid pattern in a case where the subject is irradiated with the grid-pattern measurement light LPL, it is required to accurately detect the grid pattern. For this reason, the shape of the grid-pattern measurement light LPL is not a perfect grid shape and is set to a wave shape or the like, that is, is slightly deformed from a grid shape to improve the detection accuracy of the grid pattern. Further, the grid pattern is provided with S codes indicating that the end points of the left and right horizontal line segments are continuous. In a case where the grid pattern is detected, not only the pattern but also the S codes are detected to improve the detection accuracy of the pattern. The grid pattern may be a pattern in which a plurality of spots are arranged in a grid shape in the vertical and horizontal directions in addition to a pattern in which vertical lines and horizontal lines are regularly arranged.

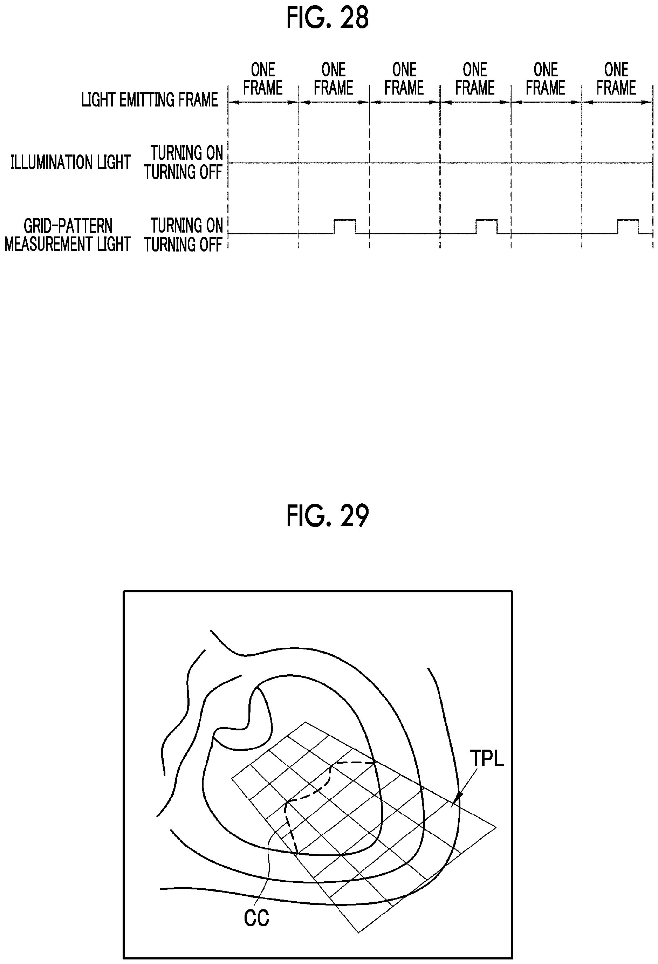

In a case where the grid-pattern measurement light LPL is used as the measurement light, in the length measurement mode, the subject may be constantly irradiated with the illumination light and the grid-pattern measurement light LPL or the subject may be constantly irradiated with the illumination light and the grid-pattern measurement light LPL may be repeatedly turned on and turned off (dimmed) every frame (or every few frames) as shown in Fig. 28 so that the subject is intermittently irradiated with the grid-pattern measurement light LPL. In this case, the three-dimensional shape is measured on the basis of the grid-pattern measurement light LPL in a frame where the grid-pattern measurement light LPL is turned on. Further, it is preferable that the measurement result of the three-dimensional shape is superimposed and displayed on an image obtained in a frame where the subject is irradiated with only the illumination light.

Three-dimensional plane light TPL, which is represented in a subject image by mesh lines as shown in Fig. 29, may be used as the measurement light (for example, see JP2017-508529A). In this case, a user moves the distal end part 12d so that the three-dimensional plane light TPL is aimed at an object to be measured. Then, in a case where the three-dimensional plane light TPL intersects with the object to be measured, a distance from an intersection curve CC between the three-dimensional plane light TPL and the subject is calculated by processing based on a manual operation of the user interface or the like, or automatic processing.

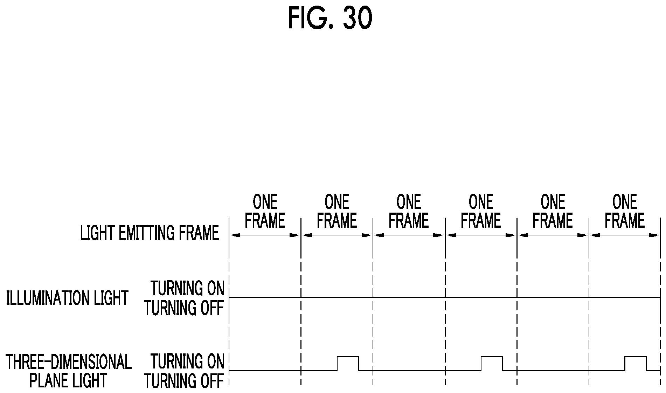

In a case where the three-dimensional plane light TPL is used as the measurement light, in the length measurement mode, the subject may be constantly irradiated with the illumination light and the three-dimensional plane light TPL or the subject may be constantly irradiated with the illumination light and the three-dimensional plane light TPL may be repeatedly turned on and turned off (dimmed) every frame (or every few frames) as shown in Fig. 30 so that the subject is intermittently irradiated with the three-dimensional plane light TPL.

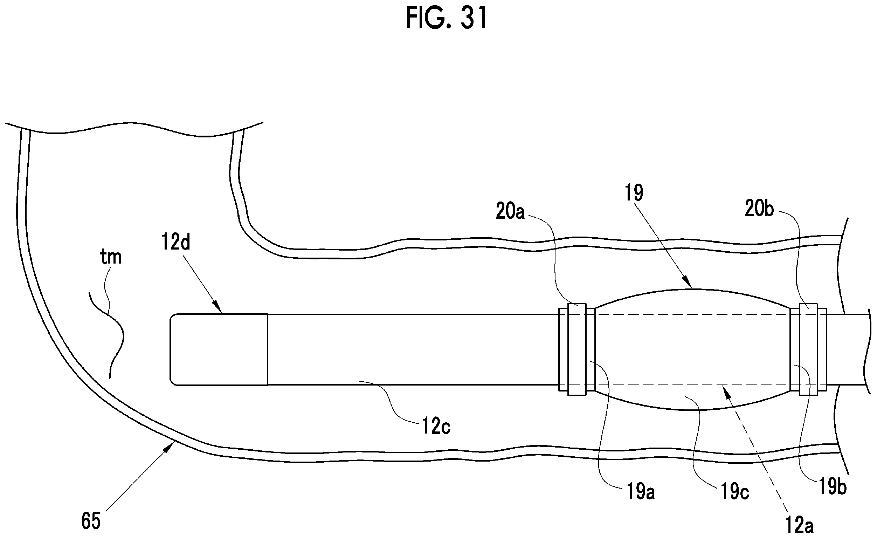

The action of the configuration will be described. In a case where the endoscope 12 is set to the length measurement mode, the system controller 41 performs a control to operate the image pickup element 32 through the image pickup controller 33 and performs a control to operate the light source unit 26 of the light source device 13 and the light source 23a of the beam light-emitting unit 23, and controls the illumination light and the auxiliary measurement light according to a preset light emission pattern. Then, after the image pickup element 32, the light source unit 26, and the light source 23a start to operate, the insertion part 12a is inserted into an object to be examined, for example, an intestinal canal 65 as shown in Fig. 31.

The balloon 19 is used to fix the position of the insertion part 12a with respect to the object to be examined as described later. However, as another use of the balloon 19, the balloon 19 may be inflated in a case where it is difficult to insert the insertion part 12a into the object to be examined, and the like. In this case, a doctor as a user operates the remote controller 16 to set the balloon 19 to the inflated state so that the inside of the object to be examined, for example, the intestinal canal 65 is widened. Then, the doctor returns the balloon 19 to the deflated state and gradually moves the insertion part 12a forward. Further, the doctor repeats an operation for gradually moving the insertion part 12a forward while setting the balloon 19 to the inflated state and the deflated state again.

The inside of the object to be examined is irradiated with light, which is emitted from the light source device 13, through the light guide 28 and the illumination lens 22a. The inside of the object to be examined is irradiated with measurement light, which is emitted from the light source 23a, through the beam light-emitting unit 23. In a state where the balloon 19 is deflated (a state shown in Fig. 31), the insertion part 12a is not fixed to the intestinal canal 65. In a case where the subject is irradiated with the measurement light and the image of the subject is picked up in a state where the balloon 19 is deflated, there is a possibility that the subject is irradiated with the measurement light and a subject image is acquired in a state where the position of the insertion part 12a of the endoscope 12 is shifted from the intestinal canal 65.

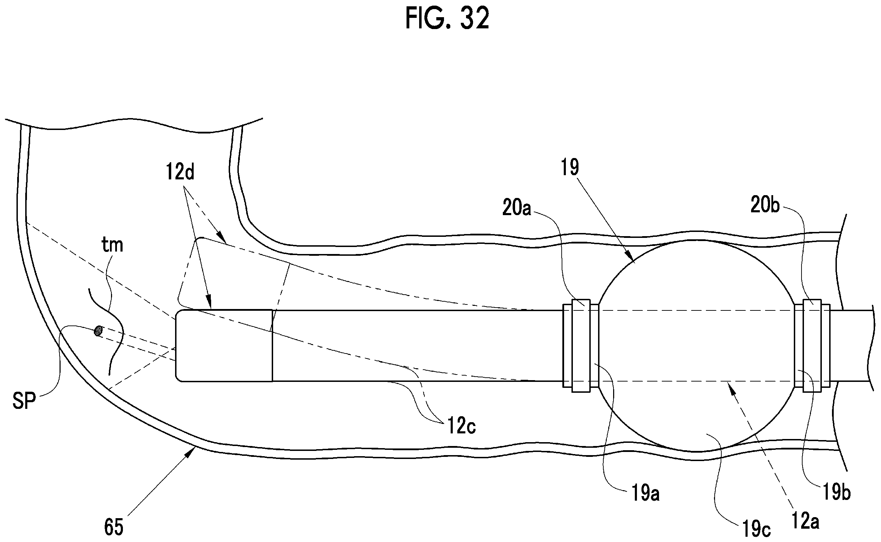

Accordingly, the user operates the instruction switch of the remote controller 16 to give an instruction to inflate the balloon 19 before the acquisition of a subject image. The balloon 19 is kept in the inflated state as shown in Fig. 32 by the control of the balloon control device 15. Since the outer diameter of the balloon 19 in the inflated state is formed depending on the inner diameter of the intestinal canal 65, the insertion part 12a is in a state where the position of the insertion part 12a is fixed in the intestinal canal 65. "A state where ∼ is fixed" mentioned here includes a state where the position of the insertion part 12a in an insertion direction is fixed but the orientation of the distal end part 12d can be finely adjusted.

Since the insertion part 12a is fixed in the intestinal canal 65, an object to be observed that the user wants to measure can be accurately irradiated with the measurement light. In a case where the user operates the freeze switch 12h in this state, the static image of the subject image is acquired. In this case, after inflating the balloon 19, the user may bend the bendable part 12c to finely adjust the orientation of the distal end part 12d.

A case where the endoscope 12 emits spotlight (measurement light) formed as a spot SP is exemplified in Fig. 32. In a state where the position of the distal end part 12d is fixed in the intestinal canal 65 as described above, for example, a tumor tm as an object to be observed is irradiated with the measurement light and a spot SP can be formed. Accordingly, it is possible to obtain an observation distance from the position of the spot SP in the subject image with high accuracy or to measure the size of the tumor tm with high accuracy.

Further, the measurement light to be emitted from the endoscope 12 is not limited to spotlight, and the same effects as described above can be obtained even in a case where measurement light, such as line-shaped light, stripe-pattern light, grid-pattern light, or three-dimensional plane light, is emitted. That is, since the insertion part 12a can be fixed by the balloon 19 even in a case where measurement light, such as line-shaped light, stripe-pattern light, grid-pattern light, or three-dimensional plane light, is emitted, measurement can be performed with high accuracy. Furthermore, since the position of the balloon 19 is closer to the proximal end than the bendable part 12c, the orientation of the distal end part 12d can be finely adjusted, that is, the visual field can be finely adjusted even though the insertion part 12a is fixed to the intestinal canal 65 by the balloon 19.

Particularly, in a case where stripe-pattern light is used as the measurement light, a subject is irradiated with the stripe-pattern light plural times while the period or phase of the stripe-pattern light is shifted by a liquid crystal shutter, a calculation is made on the basis of a plurality of images obtained by shifting the period or phase, and the three-dimensional shape of the subject is measured. In a case where the position of the insertion part 12a is shifted for the measurement of the three-dimensional shape of the subject, a phenomenon in which an image called the artifact of a moving subject or the blur of a moving subject is included occurs. However, since the position of the insertion part 12a is fixed and the same position can be irradiated with the stripe-pattern light in the present invention, the occurrence of such a phenomenon can be suppressed.

The balloon 19 attachably and detachably provided on the insertion part 12a is used in the first embodiment, but the present invention is not limited thereto. The balloon 19 and the insertion part 12a may be adapted to be integrated with each other. In this case, it is preferable that the insertion part 12a integrated with the balloon 19 is made disposable, is discarded after being used one time or a small number of times, and is replaced with a new one. The number of times mentioned here means the number of cases, and a small number of times refer to 10 times or less. Even in a case where the balloon 19 is integrated with the insertion part 12a, it is preferable that the position of the balloon 19 is closer to the proximal end than the bendable part 12c as in the first embodiment. Accordingly, even though the insertion part 12a is fixed to the intestinal canal 65 by the balloon 19, the visual field can be finely adjusted. As a configuration in which the balloon 19 and the insertion part 12a are integrated with each other, it is preferable that, for example, the balloon 19 is fixed to the insertion part 12a by an adhesive or the like. Alternatively, it is preferable that a covering layer covering the surface of the insertion part 12a and the balloon 19 are formed integrally with each other.

### [Second embodiment]

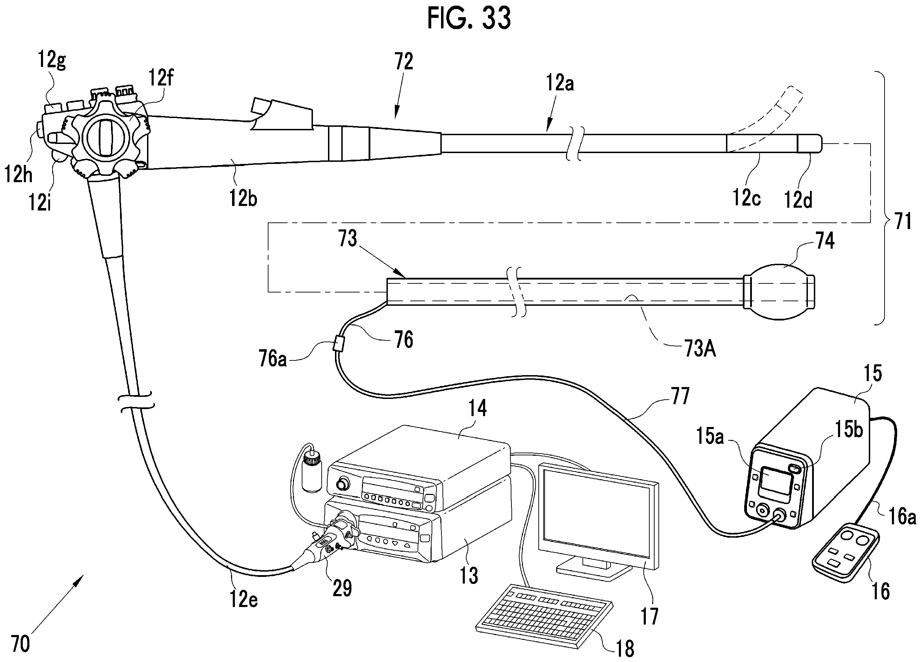

In the first embodiment, the balloon 19 is attachably and detachably provided on the insertion part 12a or is provided integrally with the insertion part 12a. However, in a second embodiment, a balloon is provided integrally with an overtube covering an insertion part. As shown in Fig. 33, an endoscope system 70 includes an endoscope 71, a light source device 13, a processor device 14, a balloon control device 15, a remote controller 16, a monitor 17, and a user interface 18. The endoscope 71 includes an endoscope body 72, an overtube 73, and a balloon 74. The same components and parts as those of the first embodiment are denoted by the same reference numerals as those of the first embodiment, and the description thereof will be omitted.

Since the endoscope body 72 does not have a configuration in which a balloon is attachable to and detachable from the insertion part 12a, the endoscope 71 has the same configuration as the endoscope 12 according to the first embodiment except that the endoscope 71 does not include the fluid pipe line 30 and the opening 31.

The overtube 73 is an overtube that covers the insertion part 12a to make the hardness of the insertion part 12a variable, and is formed in a substantially tubular shape using a material harder than the insertion part 12a, for example, silicone rubber. The material of the overtube 73 is not limited thereto, and a flexible resin, such as polyurethane, may be used.

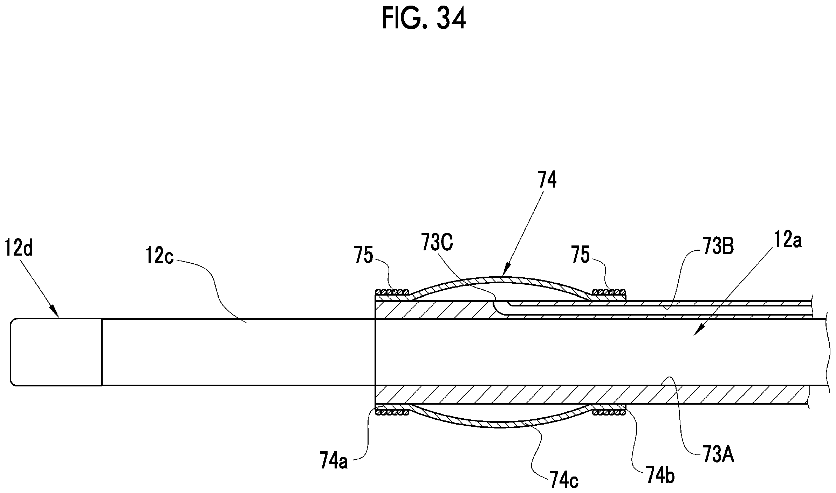

As shown in Fig. 34, the balloon 74 as a fixing member is formed in a substantially tubular shape, of which end portions are narrowed, using an elastic material, such as rubber, and includes a distal end portion 74a and a proximal end portion 74b having a small diameter and an intermediate bulging portion 74c. The balloon 74 covers the outer peripheral surface of the distal end portion of the overtube 73. For example, threads 75 are wound on the distal end portion 74a and the proximal end portion 74b and an adhesive is applied to the threads 75, so that the balloon 74 is fixed to the overtube 73.

An insertion pipe line 73A and a fluid pipe line 73B are formed in the overtube 73 in the axial direction of the overtube 73. The insertion pipe line 73A is a hole into which the insertion part 12a of the endoscope body 72 is to be inserted, and the inner diameter of the insertion pipe line 73A is slightly smaller than the outer diameter of the insertion part 12a. Accordingly, in a case where the insertion part 12a is inserted into the insertion pipe line 73A, the overtube 73 is in close contact with the outer peripheral surface of the insertion part 12a due to the elastic force of the overtube 73.

The balloon 74 is mounted on the outer peripheral surface of the distal end portion of the overtube 73 as described above. The fluid pipe line 73B is a pipe line that is used to supply fluid to the balloon 74 and to suck fluid from the balloon 74, and is provided in the pipe wall of the insertion pipe line 73A. The fluid pipe line 73B communicates with an opening 73C for the balloon that is formed on the outer peripheral surface of the overtube 73. The opening 73C is formed at the mounting position of the balloon 74, and fluid is supplied or sucked from the opening 73C, so that the balloon 74 is inflated or deflated. A tube 76 (see Fig. 34) having a small diameter is connected to the proximal end of the fluid pipe line 73B, and a connector 76a (see Fig. 34) is connected to the proximal end of the tube 76.

A tube 77 is connected to the connector 76a, and the tube 77 is connected to the balloon control device 15. The balloon control device 15 supplies and sucks fluid to inflate and deflate the balloon 74 as in the inflation and deflation of the balloon 19 of the first embodiment.

In a case where the balloon 74 is inflated, it is preferable that the outer diameter of the inflated balloon 19 is formed depending on the inner diameter of the intestinal canal of the large intestine in a case where the endoscope body 72 is, for example, a colonofiberscope as in the first embodiment. In a case where the balloon 74 is kept in the inflated state, the position of the insertion part 12a can be fixed with respect to an object to be examined as in the first embodiment.

In a case where the insertion part 12a is covered with the overtube 73, it is preferable that the position of the balloon 74 is closer to the proximal end than the bendable part 12c as in the first embodiment (a state shown in Fig. 30). Accordingly, it is possible to fine adjust the orientation of the distal end part 12d by bending the bendable part 12c after inflating the balloon 74. Further, since the overtube 73 is in close contact with the outer peripheral surface of the insertion part 12a due to the elastic force of the overtube 73 as described above, the position of the overtube 73 is not shifted with respect to the insertion part 12a.

Furthermore, since the balloon 74 fixed to the overtube 73 by adhesion is used, it is possible to obtain an effect of fixing the position of the insertion part 12a with respect to an object to be examined as in the first embodiment in a case where the overtube 73 and the balloon 74 of this embodiment are used even in a normal type endoscope not including an inflation/deflation mechanism for a balloon as in an endoscope in the related art.

The overtube 73 of the second embodiment is an overtube that is used to make the hardness of the insertion part 12a variable, but is not limited thereto. For example, an overtube used as a sliding tube can also be applied. In this case, during the use of the overtube, a lubricant, such as water, is supplied to the inner peripheral surface of the insertion pipe line (a gap between the insertion part 12a and the overtube) to reduce friction between the insertion part 12a and the overtube. Accordingly, it is possible to insert the insertion part 12a into an object to be examined while moving the insertion part 12a relative to the overtube.

The inflated and deflated states of each of the balloons 19 and 74 are controlled by the balloon control device 15 serving as an inflation/deflation device in each embodiment, but the present invention is not limited thereto. As a modification example of each embodiment, a pump, a syringe, or the like is connected to a fluid pipe line that is used to supply fluid to a balloon and to suck fluid from the balloon, and the balloon is manually inflated and deflated by a user. In this modification example, with regard to a configuration in which the balloon is inflated and deflated, the balloon may be manually inflated and deflated by a user using the pump, the syringe, or the like. However, it is preferable that the endoscope system comprises a mechanism for automatically controlling the pressure of fluid to be supplied to the balloon, for example, a mechanism for discharging a part of fluid to be supplied to the balloon to the outside in a case where the pressure of fluid exceeds a threshold value. The reason for this is to maintain the pressure of fluid to be supplied to the balloon at a constant value and to avoid a burden on an object to be examined.

In each of the embodiments, the hardware structures of processing units, which perform various types of processing, such as the reception unit 38, the signal processing unit 39, the display controller 40, the system controller 41, the irradiation region-recognizing section 58, and the measurement marker setting section 61, are various processors to be described below. Various processors include: a central processing unit (CPU) that is a general-purpose processor functioning as various processing units by executing software (program); a programmable logic device (PLD) that is a processor of which the circuit configuration can be changed after manufacture, such as a field programmable gate array (FPGA); a dedicated electrical circuit that is a processor having circuit configuration designed exclusively to perform various types of processing; and the like.

One processing unit may be formed of one of these various processors, or may be formed of a combination of two or more same kind or different kinds of processors (for example, a plurality of FPGAs, or a combination of a CPU and an FPGA). Further, a plurality of processing units may be formed of one processor. As an example where a plurality of processing units are formed of one processor, first, there is an aspect where one processor is formed of a combination of one or more CPUs and software as typified by a computer, such as a client or a server, and functions as a plurality of processing units. Second, there is an aspect where a processor fulfilling the functions of the entire system, which includes a plurality of processing units, by one integrated circuit (IC) chip as typified by System On Chip (SoC) or the like is used. In this way, various processing units are formed using one or more of the above-mentioned various processors as hardware structures.

In addition, the hardware structures of these various processors are more specifically electrical circuitry where circuit elements, such as semiconductor elements, are combined. Further, the hardware structure of the storage unit is a storage device, such as a hard disc drive (HDD), a solid state drive (SSD), or a non-volatile memory.

### Explanation of References

10: endoscope system
12: endoscope
12a: insertion part
12b: operation part
12c: bendable part
12d: distal end part
12e: universal cable
12f: angle knob
12g: mode changeover switch
12h: freeze switch
12i: zoom operation part
13: light source device
14: processor device
15: balloon control device
15a: display unit
15b: power switch
16: remote controller
16a: cable
17: monitor
18: user interface
19: balloon
19a: distal end portion
19b: proximal end portion
19c: bulging portion
20a: ring
20b: ring
21: image pickup optical system
21a: objective lens
21b: zoom lens
22: illumination optical system
22a: illumination lens
23: beam light-emitting unit
23a: light source
23b: diffractive optical element
23c: prism
23d: emitting section
24: opening
25: air/water supply nozzle
26: light source unit
27: light source controller
28: light guide
29: connector
29a: endoscope-side cap
29b: tube
30: fluid pipe line
30a: light source
31: opening
32: image pickup element
33: image pickup controller
34: CDS/AGC circuit
35: A/D converter
36: communication interface (I/F)
37: communication Interface (I/F)
38: reception unit
39: signal processing unit
40: display controller
41: system controller
42: static image storage unit
58: irradiation region-recognizing section
61: measurement marker setting section
62: marker table
65: intestinal canal
70: endoscope system
71: endoscope
72: endoscope body
73: overtube
73A: insertion pipe line
73B: fluid pipe line
73C: opening
74: balloon
74a: distal end part
74b: proximal end portion
74c: bulging portion
75: thread
76: tube
76a: connector
77: tube
80: intersection line
82: direction
101: solid line
102: dotted line
Ax: optical axis
BF: blue color filter
CC: intersection curve
D1: first direction
D2: second direction
EP: measurement point
GF: green color filter
LA: label
Lm: optical axis
LPL: measurement light
M1, M2, M3, M4, M5, M6, M7A, M7B, M7C, M8A, M8B, M8C, M9A, M9B, M9C, M10A, M10B, M10C: marker
Ma: measurement marker
Mx: gradation
P: polyp
Px: near end
Py: intermediate vicinity
Pz: far end
Qx, Qy, Qz: arrow
r1: outer diameter
RF: red color filter
Rx: range
SH: shadow
SP, SP1, SP2, SP3: spot
tm, tm1, tm2, tm3: tumor
TPL: three-dimensional plane light
ZPL: stripe-pattern light

## Claims

1. An endoscope comprising:
an insertion part that is to be inserted into an object to be examined;
a distal end part that is provided at a distal end of the insertion part;
a bendable part that is connected to a proximal end of the distal end part and changes an orientation of the distal end part by being operated to be bent;
an emitting unit that is disposed in the distal end part and emits measurement light;
an image pickup optical system that is provided in the distal end part and picks up an image of an inside of the object to be examined; and
a fixing member that fixes a position of the insertion part with respect to the object to be examined.

2. The endoscope according to claim 1,
wherein the fixing member is a balloon, and
the balloon is attachably and detachably provided on the insertion part.

3. The endoscope according to claim 1,
wherein the fixing member is a balloon, and
the balloon is provided integrally with the insertion part.

4. An endoscope comprising:
an endoscope body including an insertion part that is to be inserted into an object to be examined, a distal end part that is provided at a distal end of the insertion part, a bendable part that is connected to a proximal end of the distal end part and changes an orientation of the distal end part by being operated to be bent, an emitting unit that is disposed in the distal end part and emits measurement light, and an image pickup optical system that is provided in the distal end part and picks up an image of an inside of the object to be examined;
an overtube that covers the insertion part; and
a fixing member that fixes a position of the insertion part with respect to the object to be examined.

5. The endoscope according to claim 4,
wherein the fixing member is a balloon, and
the balloon is provided integrally with the overtube.

6. The endoscope according to any one of claims 1 to 5,
wherein the balloon is positioned closer to a proximal end of the insertion part than the bendable part.

7. The endoscope according to any one of claims 1 to 6,
wherein the measurement light is one or a plurality of spotlights.

8. The endoscope according to any one of claims 1 to 6,
wherein the measurement light is line-shaped measurement light.

9. The endoscope according to any one of claims 1 to 6,
wherein the measurement light is grid-pattern measurement light.

10. The endoscope according to any one of claims 1 to 6,
wherein the measurement light is three-dimensional plane light.

11. The endoscope according to any one of claims 7 to 10,
wherein the subject is intermittently irradiated with the measurement light.

12. The endoscope according to any one of claims 1 to 6,
wherein the measurement light is stripe-pattern light.

13. The endoscope according to claim 12,
wherein the subject is irradiated with a plurality of types of the stripe-pattern light having different phases or periods while the plurality of types of the stripe-pattern light are switched.

14. An endoscope system comprising:
an endoscope including an insertion part that is to be inserted into an object to be examined, a distal end part that is provided at a distal end of the insertion part, a bendable part that is connected to a proximal end of the distal end part and changes an orientation of the distal end part by being operated to be bent, an emitting unit that is disposed in the distal end part and emits measurement light, an image pickup optical system that is provided in the distal end part and picks up an image of an inside of the object to be examined, and a balloon that fixes a position of the insertion part with respect to the object to be examined; and
an inflation/deflation device that supplies fluid to the balloon and sucks the fluid from the balloon to inflate or deflate the balloon.
